(19) Europäisches Patentamt
European Patent Office
Office européen des brevets

(11) **EP 2 329 008 B1**

(12) **FASCICULE DE BREVET EUROPEEN**

(45) Date de publication et mention de la délivrance du brevet:
**28.06.2017 Bulletin 2017/26**

(51) Int Cl.:
*C12N 1/18* (2006.01)   *C12R 1/865* (2006.01)

(21) Numéro de dépôt: **09740346.3**

(22) Date de dépôt: **10.09.2009**

(86) Numéro de dépôt international:
**PCT/FR2009/001080**

(87) Numéro de publication internationale:
**WO 2010/031916 (25.03.2010 Gazette 2010/12)**

(54) **NOUVELLES SOUCHES DE LEVURE POUR LA PRODUCTION D'ALCOOL**

NEUE HEFE STÄMME ZUR HERSTELLUNG VON ALKOHOL

NOVEL YEAST STRAINS FOR PRODUCING ALCOHOL

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **16.09.2008 FR 0805056**

(43) Date de publication de la demande:
**08.06.2011 Bulletin 2011/23**

(73) Titulaire: **Lesaffre et Compagnie
75001 Paris (FR)**

(72) Inventeurs:
• **COLAVIZZA, Didier**
**59100 Roubaix (FR)**
• **TOUSSAINT, Renaud**
**59147 Gondecourt (FR)**

(74) Mandataire: **Cabinet Plasseraud
66 rue de la Chaussée d'Antin
75440 Paris Cedex 09 (FR)**

(56) Documents cités:
**EP-A1- 0 798 382     WO-A-98/31784**

• **BLASCO LUCIA ET AL: "Genetic stabilization of Saccharomyces cerevisiae oenological strains by using benomyl" INTERNATIONAL MICROBIOLOGY, vol. 11, no. 2, juin 2008 (2008-06), pages 127-132 URL, XP009115209 ISSN: 1139-6709**

• **ROMANO P ET AL: "IMPROVEMENT OF A WINE SACCHAROMYCES-CEREVISIAE STRAIN BY A BREEDING PROGRAM" APPLIED AND ENVIRONMENTAL MICROBIOLOGY, vol. 50, no. 4, 1985, pages 1064-1067, XP002912393 ISSN: 0099-2240**

• **CARRO DAVID ET AL: "Genetic analysis of the karyotype instability in natural wine yeast strains" YEAST, vol. 18, no. 16, décembre 2001 (2001-12), pages 1457-1470, XP002523952 ISSN: 0749-503X**

• **MARULLO P ET AL: "Inheritable nature of enological quantitative traits is demonstrated by meiotic segregation of industrial wine yeast strains" FEMS YEAST RESEARCH, vol. 4, no. 7, mai 2004 (2004-05), pages 711-719, XP002523953 ISSN: 1567-1356**

• **SIPICZKI M ET AL: "Genetic segregation of natural Saccharomyces cerevisiae strains derived from spontaneous fermentation of Aglianico wine." JOURNAL OF APPLIED MICROBIOLOGY 2004, vol. 96, no. 5, 2004, pages 1169-1175, XP002523954 ISSN: 1364-5072**

• **MIKLOS I ET AL: "BREEDING OF A DISTILLER'S YEAST BY HYBRIDIZATION WITH A WINE YEAST" APPLIED MICROBIOLOGY AND BIOTECHNOLOGY, vol. 35, no. 5, 1991, pages 638-642, XP009115217 ISSN: 0175-7598**

- SHINOHARA T ET AL: "Selection and hybridization of wine yeasts for improved winemaking properties: Fermentation rate and aroma productivity", JOURNAL OF FERMENTATION AND BIOENGINEERING, SOCIETY OF FERMENTATION TECHNOLOGY, JP, vol. 77, no. 4, 1 January 1994 (1994-01-01), pages 428-431, XP025748753, ISSN: 0922-338X, DOI: 10.1016/0922-338X(94)90018-3 [retrieved on 1994-01-01]
- DATABASE WPI Week 199342, Derwent Publications Ltd., London, GB; AN 1993-330570 & JP H05 236 942 A (TSUSHO SANGYOSHO KISOSANGYO KYOKUCHO) 17 Septembre 1993
- DATABASE WPI Week 198750, Derwent Publications Ltd., London, GB; AN 1987-353770 & SU 1 306 949 A (GEN GENETICS INST) 30 Avril 1987
- DATABASE WPI Week 200911, Derwent Publications Ltd., London, GB; AN 2009-B40424 & RU 2 340 666 C1 (BALTIKA BEER-BREWING CO) 10 Décembre 2008

**Description**

DOMAINE TECHNIQUE

La présente invention concerne de nouvelles souches de levure et des levures issues de ces nouvelles souches destinées à la production d'alcool, ainsi qu'un procédé d'obtention de telles souches.

ARRIERE-PLAN TECHNOLOGIQUE

Les levures subissent fréquemment des mutations lors de leur multiplication et lors de leur utilisation en fermentation, en particulier à l'échelle industrielle. Toutefois, ces levures conservent généralement un phénotype normal, parce qu'il s'agit souvent de mutations récessives et que les souches industrielles sont habituellement aneuploïdes ou polyploïdes (The Alcohol Textbook, Ed Jacques (2003)).

Le recyclage des levures est souvent utilisé en fermentation alcoolique. Le recyclage des levures consiste en plusieurs utilisations successives des levures, sans repartir des levures ou de la souche de départ. Ainsi, tout ou partie des levures issues d'une fermentation i est utilisé pour une fermentation ultérieure i+1 ; puis tout ou partie des levures issues de la fermentation i+1 est utilisé pour une fermentation i+2, et ainsi de suite.

Le recyclage des levures, parfois appelé « repiquage en série » dans la littérature, est une technique qui expose la levure à un grand nombre de stress pouvant entraîner des mutations de son patrimoine génétique et des dérives génétiques.

Dans leurs travaux, Powell et Diacetis (2007, J. Inst. Brew. 1131(1), 67-74) étudient l'impact du recyclage des levures de brasserie sur leur phénotype. A la fin de chaque fermentation, une partie de la biomasse de levures est prélevée pour effectuer la fermentation suivante. Powell et Diacetis n'ont observé aucune altération dans les caractéristiques de fermentation des levures au cours des repiquages successifs, sur environ 135 générations.

Verma et al. (1983) ont par ailleurs étudié l'intérêt du recyclage de différentes levures de *Saccharomyces cerevisiae* dans le cadre de la production d'éthanol. Après chaque fermentation alcoolique, les levures sont repiquées en vue d'une nouvelle fermentation, et la quantité d'alcool produit à l'issue de chaque fermentation est mesurée. Les auteurs ont mis en évidence plusieurs souches de levure capables de subir 6 à 10 repiquages successifs sans que leur production d'alcool soit sensiblement altérée.

Ainsi, dans la littérature, la stabilité des levures en terme de production d'alcool a donc été étudiée dans le cadre du recyclage des levures en fermentation alcoolique.

Lorsqu'elle est multipliée dans un procédé industriel classique, la levure la plus performante, en terme de production d'alcool, connue de la Société Demanderesse montre cependant parfois des diminutions considérables de la quantité d'alcool produit. Or, la multiplication des levures à l'échelle industrielle à partir d'une souche de levure doit aboutir à une levure de qualité fiable. Par « qualité fiable », on entend ici que la quantité d'alcool produit par la levure, dans un schéma de fermentation alcoolique donné, est relativement constante.

Par ailleurs, le marché de la levure destinée à la production d'alcool est toujours demandeur de levures plus performantes en terme de quantité d'alcool produit.

Il y a donc un réel besoin de fournir de nouvelles souches de levure donnant des levures de qualité fiable, et de préférence performantes en terme de quantité d'alcool produit.

RESUME DE L'INVENTION

Un premier objet de l'invention est de fournir de nouvelles souches de levure stables.

La présente invention a ainsi pour objet une souche de *Saccharomyces cerevisiae* stable choisie parmi la souche déposée à la CNCM sous le numéro I-4073, la souche déposée à la CNCM sous le numéro I-4074 ou la souche déposée à la CNCM sous le numéro I-4075.

Un deuxième objet de l'invention est de fournir un nouveau procédé d'obtention de souches de levure stables, ledit procédé comprenant les étapes de :

- croisement d'une souche de *Saccharomyces* instable avec elle-même ou avec une autre souche de *Saccharomyces*, pour obtenir au moins un hybride,
- culture d'au moins un hybride, pour obtenir une population de levure R0,
- réalisation de n repiquages successifs de la population de levure R0, n étant un nombre entier supérieur ou égal à 6, pour obtenir une population de levures Rn,
- mesure de la production d'alcool de ladite population R0 et celle de la production d'alcool de ladite population Rn,
- sélection d'au moins un hybride pour lequel la production d'alcool de la population Rn est supérieure ou égale à 95% de celle de la population R0, pour obtenir des souches de levure stables.

La présente invention a particulièrement pour objet un procédé d'obtention de souches de levure stables tel que défini ci-dessus, comprenant une étape supplémentaire de sélection d'au moins un hybride pour lequel la production d'alcool de la population R0 est supérieure ou égale à 95% de celle d'une population R0 de la souche déposée à la CNCM le 4 septembre 2008 sous le numéro I-4072.

La présente invention a également pour objet un procédé d'obtention de souches de levure stables tel que défini ci-dessus, comprenant une étape supplémentaire de sélection d'au moins un hybride ayant un rendement de biomasse supérieur ou égal à 90% du rendement de biomasse de la souche I-4072.

La présente invention a également pour objet un procédé d'obtention de souches de levure stables tel que défini ci-dessus, caractérisé en ce que l'étape de croisement de souches de levure comprend les étapes suivantes :

- sporulation de ladite souche de *Saccharomyces* instable et éventuellement de ladite autre souche de *Saccharomyces*, pour obtenir des ségrégeants,
- culture de chaque ségrégeant, pour obtenir une population R0 pour chaque ségrégeant,
- réalisation de m repiquages successifs de chaque ségrégeant, pour obtenir une population Rm de chaque ségrégeant, m étant un nombre entier supérieur ou égal à 6,
- mesure de la production d'alcool de la population R0 et celle de la production d'alcool de la population Rm de chaque ségrégeant,
- sélection des ségrégeants pour lesquels :

  ◦ la production d'alcool de la population Rm est supérieure ou égale à 70% de celle de la population R0 dudit ségrégeant et
  ◦ la production d'alcool de la population Rm est supérieure ou égale à 85% de celle d'une population R0 de la souche I-4072,

  pour obtenir des ségrégeants stables et performants, et
- croisement de ségrégeants stables et performants issus de ladite souche instable avec des ségrégeants stables et performants de signe opposé issus de ladite souche instable ou de ladite autre souche, pour obtenir au moins un hybride.

La présente invention a particulièrement pour objet un procédé d'obtention de souches de levure stables tel que défini ci-dessus, dans lequel ladite souche de levure instable est la souche I-4072.

La présente invention a également pour objet un procédé d'obtention de souches de levure stables tel que défini ci-dessus, dans lequel ladite autre souche est choisie parmi la souche I-4071, la souche I-4073, la souche I-4074 ou la souche I-4075.

Un troisième objet de l'invention concerne une souche de levure susceptible d'être obtenue par le procédé d'obtention de souches de levure stables tel que défini ci-dessus.

Un quatrième objet de l'invention est de fournir une souche de *Saccharomyces cerevisiae* dérivée d'une souche telle que définie ci-dessus, caractérisée en ce que :

- la production d'alcool d'une population Rn de ladite souche dérivée est supérieure ou égale à 95% de la production d'alcool d'une population R0 de ladite souche dérivée, et/ou
- la production d'alcool d'une population R0 de ladite souche dérivée est supérieure ou égale à 95% de la production d'alcool d'une population R0 de la souche déposée à la CNCM sous le numéro I-4072,

la population Rn étant issue de n repiquages successifs de la population R0, n étant un nombre entier supérieur ou égal à 6.

Un cinquième objet de l'invention est de fournir un nouveau procédé d'évaluation de la stabilité d'une souche de levure, comprenant les étapes de :

- culture de ladite souche, pour obtenir une population de levure R0,
- réalisation de n repiquages successifs de la population de levure R0, n étant un nombre entier supérieur ou égal à 6, pour obtenir une population de levures Rn,
- mesure de la production d'alcool de ladite population R0 et celle de la production d'alcool de ladite population Rn,
- qualification de ladite souche de « stable » si la production d'alcool de la population Rn est supérieure ou égale à 95% de la production d'alcool de la population R0.

Un sixième objet de l'invention concerne une levure obtenue par culture d'une souche de levure telle que définie ci-dessus.

La présente invention a particulièrement pour objet une levure telle que définie ci-dessus, caractérisée en ce que sa production d'alcool est supérieure ou égale à 95% de la production d'alcool d'une levure issue de la souche I-4072.

La présente invention a également pour objet une levure telle que définie ci-dessus, caractérisée en ce qu'elle est sous forme de crème de levure, de levure pressée, de levure sèche ou de levure surgelée.

Un septième objet de l'invention concerne l'utilisation d'une levure telle que définie ci-dessus pour produire de l'alcool.

## DESCRIPTION DETAILLEE DE L'INVENTION

La présente invention a pour objet de fournir de nouvelles souches de *Saccharomyces cerevisae* et de nouvelles levures issues de ces souches destinées à la production d'alcool, lesdites levures étant de qualité fiable et, de préférence, performantes en terme de quantité d'alcool produit.

Pour obtenir une levure de qualité fiable, il faut disposer d'une souche de levure stable.

Une souche de levure stable désigne ici une souche qui donne des levures dont la production d'alcool est relativement stable au fil des générations.

Pour obtenir une levure performante, en terme de quantité d'alcool produit, il faut disposer d'une souche de levure performante en terme de quantité d'alcool produit.

Les expressions «production d'alcool », «production d'éthanol », «quantité d'alcool produit » et « quantité d'éthanol produit » sont ici synonymes.

Toute souche qui ne répond pas à la définition d'une souche stable est qualifiée de souche instable.

De manière surprenante et inattendue, la Société Demanderesse a démontré qu'au cours de la multiplication des levures à l'échelle industrielle, à partir de la souche I-4072, apparaissent des variants dont la production d'alcool est faible. Au bout d'un certain nombre de générations, la proportion croissante de variants se traduit par une diminution considérable de la quantité d'alcool produit.

Or, ce problème de stabilité en terme de production d'alcool, qui apparaît au cours de la multiplication des levures, n'a jamais été mis en évidence dans la littérature.

En effet, les documents de l'art antérieur abordent le problème dé stabilité des souches uniquement dans le cadre du recyclage des levures entre plusieurs fermentations alcooliques.

Il y avait donc un besoin de fournir un procédé permettant de distinguer les souches de levure stables des souches de levure instables.

Afin de pouvoir vérifier qu'une souche de levure est stable, en terme de production d'alcool, la Société Demanderesse a élaboré un nouveau procédé permettant d'évaluer le caractère stable d'une souche.

Ce procédé original d'évaluation de la stabilité d'une souche de levure est basé sur la réalisation de plusieurs repiquages successifs à partir de la souche à évaluer, afin d'obtenir une multiplication des levures mimant celle obtenue lors d'une multiplication à l'échelle industrielle.

Le procédé d'évaluation de la stabilité d'une souche de levure selon l'invention comporte une combinaison originale de deux étapes :

- une première étape consistant en plusieurs repiquages successifs à partir de la souche de départ, et
- une seconde étape de comparaison de la production d'alcool de la population de levures obtenue à l'issue des repiquages successifs avec celle de la souche de départ à évaluer.

Ainsi, si la production d'alcool de la population de levures obtenue à l'issue des repiquages n'a pas sensiblement diminué par rapport à la production d'alcool de la souche de départ à évaluer, la souche est qualifiée de souche stable.

La première étape de repiquages successifs aboutit, de préférence, à un nombre de générations de levures supérieur ou égal à celui obtenu à l'issue d'une multiplication des levures à l'échelle industrielle.

Le procédé d'évaluation de la stabilité d'une souche selon l'invention présente de nombreux avantages. Il s'agit en particulier d'un procédé rapide, donnant des résultats fiables, et pouvant être mis en oeuvre au laboratoire.

La mise en oeuvre du procédé selon l'invention procure un avantage considérable pour un industriel, à la fois en terme de temps, de coût, et de qualité de ses produits, en évitant notamment de réaliser plusieurs multiplications industrielles d'une souche instable.

La présente invention a ainsi pour objet un procédé d'évaluation de la stabilité d'une souche de levure, comprenant les étapes de :

- culture de ladite souche, pour obtenir une population de levure R0,
- réalisation de n repiquages successifs de la population de levure R0, n étant un nombre entier supérieur ou égal à 6, pour obtenir une population de levures Rn,
- mesure de la production d'alcool de ladite population R0 et celle de la production d'alcool de ladite population Rn,
- qualification de ladite souche de « stable » si la production d'alcool de la population Rn est supérieure ou égale à 95% de la production d'alcool de la population R0.

L'expression « souche de levure » désigne une population relativement homogène de cellules de levure.

Une souche de levure est obtenue à partir de l'isolement d'un clone, un clone étant une population de cellules obtenue à partir d'une seule cellule de levure.

La souche à évaluer est notamment une souche destinée à la production de levures utilisées en distillerie et/ou en vinification et/ou en brasserie et/ou pour la production de boissons fermentées.

La souche à évaluer est de préférence une souche de *Saccharomyces*, notamment une souche de *Saccharomyces cerevisiae*.

Le procédé selon l'invention n'est pas limité à l'évaluation de la stabilité de souches de levure. Il peut également être appliqué à des levures.

Le terme « levures » désigne des levures obtenues par culture à partir d'une souche de levure.

La culture de la souche de levure à évaluer est effectuée dans un milieu de culture approprié à la croissance de la souche de levure.

L'homme du métier est capable de déterminer quelle est la composition d'un milieu approprié à une souche de levure donnée.

La culture de la souche de levure peut être effectuée en milieu liquide ou sur un milieu gélosé.

La culture de la souche de levure est, de préférence, effectuée sur un milieu gélosé.

La culture de la souche de levure est généralement effectuée à une température comprise de 25°C à 35°C, de préférence de 28°C à 32°C.

La culture de la souche de levure est généralement effectuée à une température de 30°C.

La culture de la souche de levure à évaluer aboutit à une population de levures appelée R0.

Ensuite, n repiquages successifs sont réalisés à partir de la population R0. Cela signifie que tout ou partie de la culture correspondant à la population de levures R0 est ensemencé pour la culture suivante qui aboutit à une population de levures G1 ; puis tout ou partie de la culture correspondant à la population de levures G1 est ensemencé pour la culture suivante qui aboutit à une population de levures G2, et ainsi de suite jusqu'à la culture qui aboutit à une population de levures Rn.

Les n repiquages successifs sont, de préférence, effectués à intervalles de temps réguliers.

De préférence, les n repiquages successifs sont des repiquages journaliers.

Dans un mode de réalisation préféré, les n repiquages sont réalisés dans le même milieu et/ou à la même température et/ou avec la même quantité de biomasse ensemencée.

La biomasse est définie comme la masse d'une population de levures.

Par exemple, lors de chaque repiquage, une biomasse correspondant à une masse sèche de 0,05 mg de levures est ensemencée, de préférence en surface d'un milieu gélosé.

Si besoin, la biomasse récoltée à l'issue d'un repiquage peut être conservée à 4°C pendant plusieurs jours, par exemple 1 à 2 jours, avant d'être ensemencée pour le repiquage suivant.

Les repiquages successifs peuvent être effectués comme indiqué dans l'exemple 1.

Le procédé d'évaluation de la stabilité d'une souche comporte un nombre de repiquages supérieur ou égal à 6, de préférence supérieur ou égal à 10, plus préférentiellement supérieur ou égal à 12, plus préférentiellement encore supérieur ou égal à 14.

Dans un mode de réalisation préféré, le procédé d'évaluation selon l'invention comporte 12 repiquages successifs.

Il est possible de déterminer le nombre de générations de levures obtenu après n repiquages successifs, en calculant le nombre de générations obtenu entre chacun des n repiquages (cf. « Yeast Physiology and Biotechnology », 1998, Graeme M. Walker, publié par Wiley, p143).

Le nombre de générations ng entre deux repiquages successifs i et i+1 est obtenu en :

- mesurant la biomasse ($X_i$) ensemencée pour le $i^{ème}$ repiquage et la biomasse ($X_{i+1}$) récoltée à la fin du $i^{ème}$ repiquage, puis

- en appliquant la formule suivante :

$$n_g = 3,3 \log (X_{i+1}/ X_i)$$

La biomasse X peut être exprimée en nombre de cellules de levure ou en poids de matière sèche.

Le nombre de cellules de levure est déterminé par les méthodes classiques bien connues de l'homme du métier, tels que le dénombrement au microscope ou la cytométrie en flux.

Le poids de matière sèche est déterminé par les méthodes classiques bien connues de l'homme du métier.

Le nombre total de générations obtenu après n repiquages successifs correspond à l'addition du nombre de générations obtenu entre chaque repiquage.

A titre d'exemple, le nombre de générations obtenu sur 12 repiquages successifs journaliers est de l'ordre d'environ 60 générations.

On obtient ainsi un nombre de générations supérieur ou égal à celui obtenu à l'issue d'une multiplication des levures à l'échelle industrielle qui est généralement de l'ordre d'environ 50 à environ 60 générations.

Ce procédé original d'évaluation de la stabilité d'une souche de levure permet ainsi d'obtenir, à l'échelle du laboratoire, un nombre de générations supérieur ou égal à celui obtenu lors d'une multiplication industrielle de levures.

De préférence, les conditions du procédé d'évaluation de la stabilité d'une souche permettent d'obtenir au moins 30 générations, de préférence au moins 40 générations, plus préférentiellement au moins 50 générations, plus préférentiellement encore au moins 60 générations.

La mesure de la production d'alcool est effectuée par tout moyen approprié connu de l'homme du métier. Il peut s'agir d'une mesure directe de l'éthanol produite ou d'une mesure indirecte via un paramètre corrélé à la production d'alcool.

Par exemple, la production d'alcool peut être mesurée par chromatographie, notamment par HPLC (*High Performance Liquid Chromatography*), un kit enzymatique (par exemple le kit de dosage de l'éthanol de Boehringer), ou un dosage par le dichromate de potassium.

La production d'alcool est de préférence mesurée dans un test de fermentation alcoolique tel que décrit dans l'exemple 1.

Dans un tel test, la souche de levure est multipliée en milieu liquide, de préférence en deux temps : une étape de pré-culture suivie d'une étape de culture. La fermentation alcoolique est ensuite réalisée, par exemple à partir de 1 g de matière sèche de levure, à 35°C, en condition anaérobie, de préférence sous agitation.

Le substrat pour la fermentation alcoolique est de préférence sous forme de saccharose présent initialement dans le milieu de culture, par exemple à une concentration de 240 g/L.

La mesure de la production d'alcool est généralement exprimée en pourcentage volumique.

La courbe de fermentation alcoolique représentant la quantité d'alcool produit en fonction du temps comporte généralement trois phases :

- une phase de latence, au cours de laquelle il n'y a pas de production d'éthanol,
- une phase exponentielle, et
- une phase de plateau, cette phase correspondant à la quantité maximale d'éthanol produit lors de cette fermentation alcoolique.

La production d'alcool peut être mesurée à un temps (t) se situant en fin de phase exponentielle ou pendant la phase de plateau.

La mesure de la production d'alcool peut être effectuée à $t_1$ et/ou $t_2$, tels qu'ils sont définis ci-dessous.

La mesure à $t_1$ se situe en fin de phase exponentielle, à un temps supérieur ou égal à celui qui correspond à 95% de la quantité maximale d'alcool produit.

Cette mesure à $t_1$ permet notamment d'apprécier la cinétique de production d'alcool.

La mesure à $t_2$ se situe pendant la phase de plateau, c'est-à-dire lorsque la fermentation alcoolique est finie.

La fin de la fermentation alcoolique correspond au moment où il n'y a plus de sucre résiduel dans le milieu pouvant être fermenté par la levure.

Cette mesure à $t_2$ permet également d'apprécier le volume maximum d'alcool produit.

A titre d'exemple, dans les conditions définies dans les exemples, la production d'alcool peut être mesurée à $t_1$=48h et/ou à $t_2$=72h.

La dernière étape du procédé d'évaluation de la stabilité d'une souche consiste à qualifier de souche stable une souche dont la production d'alcool de sa population Rn est supérieure ou égale à 95% de la production d'alcool de sa population R0.

La souche est notamment qualifiée de souche stable lorsque la production d'alcool de la population Rn de ladite souche est supérieure ou égale à 97%, de préférence supérieure ou égale à 99% de la production d'alcool de sa population R0.

Une souche est qualifiée de souche instable lorsque la production d'alcool de la population Rn de ladite souche est inférieure à 95% de la production d'alcool de sa population R0.

La mise en oeuvre du procédé d'évaluation de la stabilité d'une souche selon l'invention permet de confirmer que la souche I-4072 est une souche instable : la production d'alcool de sa population R12 est égale à 49% à $t_1$=48h et à 46,9% à $t_2$=72h de la production d'alcool de sa population R0 (voir exemple 1).

Par ailleurs, la Société Demanderesse a également mis au point un nouveau procédé original d'obtention de souches de levure stables.

La mise en oeuvre de ce procédé a en particulier permis d'obtenir les trois nouvelles souches de *Saccharomyces cerevisiae* déposées le 4 septembre 2008 en vertu du traité de Budapest auprès de la CNCM (Collection Nationale de Cultures de Micro-organismes, Institut Pasteur, 25 rue du Docteur Roux, F-75724 Paris Cedex 15, France) sous les numéro I-4073, I-4074 et I-4075.

La présente invention a ainsi pour objet une souche de *Saccharomyces cerevisiae* choisie parmi la souche déposée à la CNCM sous le numéro I-4073, la souche déposée à la CNCM sous le numéro I-4074 ou la souche déposée à la CNCM sous le numéro I-4075.

Ces trois souches de levure sont à la fois stables et performantes.

La stabilité de ces souches de levure se traduit par le faible écart dans la quantité d'alcool produit, après n repiquages successifs, par rapport à la souche de départ.

L'évaluation de la stabilité des souches selon l'invention peut être effectuée comme décrit ci-dessus dans le procédé d'évaluation de la stabilité d'une souche.

La stabilité des trois souches selon l'invention se traduit ainsi par une diminution de la production d'alcool inférieure à 3% après n repiquages, alors qu'une souche instable comme la souche de référence I-4072 montre une diminution de la production d'alcool supérieur à 50% (voir exemple 1).

La performance des souches de levure selon l'invention se traduit par une production d'alcool desdites souches voisine de celle de la souche de référence I-4072.

La performance des trois souches de levure selon l'invention se traduit ainsi par une production d'alcool desdites souches supérieure ou égale à 95% de celle de la souche de référence.

La souche de *Saccharomyces cerevisiae* déposée le 4 septembre 2008 auprès de la CNCM sous le numéro I-4075 présente notamment les caractéristiques suivantes :

- la production d'alcool de la population R12 de ladite souche est supérieure ou égale à 97% de la production d'alcool de la population R0 de ladite souche, la population R12 étant issue de 12 repiquages successifs journaliers de la population R0, et
- la production d'alcool de la population R0 de ladite souche est supérieure ou égale à 99% de la production d'alcool de la population R0 de la souche déposée à la CNCM sous le numéro I-4072 (lors d'une mesure à 48h).

La souche de *Saccharomyces cerevisiae* déposée le 4 septembre 2008 auprès de la CNCM sous le numéro I-4073 présente notamment les caractéristiques suivantes :

- la production d'alcool de la population R12 de ladite souche est supérieure ou égale à 98% de la production d'alcool de la population R0 de ladite souche, la population R12 étant issue de 12 repiquages successifs journaliers de la population R0, et
- la production d'alcool de la population R0 de ladite souche est supérieure ou égale à 99% de la production d'alcool de la population R0 de la souche déposée à la CNCM sous le numéro I-4072.

La souche de *Saccharomyces cerevisiae* déposée le 4 septembre 2008 auprès de la CNCM sous le numéro I-4074 présente notamment les caractéristiques suivantes :

- la production d'alcool de la population R12 de ladite souche est supérieure ou égale à 98% de la production d'alcool de la population R0 de ladite souche, la population R12 étant issue de 12 repiquages successifs journaliers de la population R0, et
- la production d'alcool de la population R0 de ladite souche est supérieure ou égale à 99% de la production d'alcool de la population R0 de la souche déposée à la CNCM sous le numéro I-4072.

La présente invention a donc pour objet les trois souches décrites ci-dessus et l'ensemble des souches appartenant à la même famille, en particulier toutes les souches dérivées qui partagent les mêmes propriétés que ces trois souches.

Par ailleurs, la présente invention a pour objet un nouveau procédé original d'obtention de souches de levure stables, basé sur la sélection de nouvelles souches qui répondent à la qualification de souche stable dans le procédé d'évaluation de la stabilité d'une souche tel que défini ci-dessus.

Ladite sélection est effectuée sur des nouvelles souches de levure obtenues par des techniques classiques.

Une technique classique consiste en une étape de sporulation de levures pour obtenir des ségrégeants, suivie d'une étape de croisement des ségrégeants pour obtenir des hybrides. Les hybrides constituent les souches de levures qui sont par la suite sélectionnées sur le critère de stabilité.

La présente invention a ainsi pour objet un procédé d'obtention de souches de levure stables, ledit procédé comprenant les étapes de :

- croisement d'une souche de *Saccharomyces* instable avec elle-même ou avec une autre souche de *Saccharomyces,* pour obtenir au moins un hybride,
- culture d'au moins un hybride, pour obtenir une population de levure R0,
- réalisation de n repiquages successifs de la population de levure R0, n étant un nombre entier supérieur ou égal à 6, pour obtenir une population de levures Rn,
- mesure de la production d'alcool de ladite population R0 et celle de la production d'alcool de ladite population Rn,
- sélection d'au moins un hybride pour lequel la production d'alcool de la population Rn est supérieure ou égale à 95% de celle de la population R0, pour obtenir des souches de levure stables.

L'étape de croisement est réalisée selon les techniques classiques, comme celles enseignées dans le chapitre 7 « Sporulation and Hydridization of Yeast » par R.R. Fowell, de l'ouvrage de référence « The Yeasts », Volume 1, édité par A.H. Rose et J. S. Harrison, 1969-Academic Press.

Les étapes de culture d'au moins un hybride, réalisation de n repiquage successifs, et mesure de la production d'alcool sont similaires à celles du procédé d'évaluation de la stabilité d'une souche.

Les conditions de mise en oeuvre de ces étapes sont donc les mêmes que celles décrites ci-dessus dans le cadre du procédé d'évaluation de la stabilité d'une souche.

La dernière étape du procédé d'obtention de souches de levure stables consiste à sélectionner au moins un hybride pour lequel la production d'alcool de la population Rn est supérieure ou égale à 95% de celle de la population R0, un tel hybride constituant une souche de levure stable.

De préférence, la dernière étape du procédé d'obtention de souches de levure stables consiste à sélectionner au moins un hybride pour lequel la production d'alcool de la population Rn est supérieure ou égale à 97% de celle de la population R0.

La présente invention a particulièrement pour objet un procédé d'obtention de souches de levure stables tel que défini ci-dessus, dans lequel la dernière étape de sélection comprend la sélection d'au moins un hybride pour lequel la production d'alcool de la population Rn est supérieure ou égale à 97% de celle de la population R0, de préférence supérieure ou égale à 99% de celle de la population R0.

Les souches stables selon l'invention, la souche instable utilisée à l'étape de croisement et ladite autre souche de *Saccharomyces* utilisée à l'étape de croisement peuvent être des souches homothalliques ou hétérothalliques.

Dans un mode de réalisation préféré de l'invention, les souches stables selon l'invention, la souche instable utilisée à l'étape de croisement et ladite autre souche de *Saccharomyces* utilisée à l'étape de croisement sont des souches hétérothalliques.

La présente invention a également pour objet un procédé d'obtention de souches de levure à la fois stables et performantes en terme de production d'alcool.

La présente invention a ainsi pour objet un procédé d'obtention de souches de levure stables tel que défini ci-dessus, comprenant une étape supplémentaire de sélection d'au moins un hybride pour lequel la production d'alcool de la population R0 est supérieure ou égale à 95% de celle de la population R0 de la souche I-4072.

Cette étape supplémentaire de sélection sur la base de la performance en terme de production d'alcool est effectuée avant ou après l'étape de sélection des hybrides sur le critère de stabilité.

La présente invention a particulièrement pour objet un procédé d'obtention de souches de levure stables tel que défini ci-dessus, dans lequel l'étape supplémentaire de sélection comprend la sélection d'au moins un hybride pour lequel la production d'alcool de la population R0 est supérieure ou égale à 97%, de préférence supérieure ou égale à 99% de celle de la population R0 de la souche I-4072.

La présente invention a également pour objet un procédé d'obtention de souches de levure à la fois stables et performantes en terme de production d'alcool et ayant un bon rendement de biomasse.

Par « rendement » ou « rendement de biomasse » ou « rendement de production de biomasse », on désigne ici le rapport de la masse de levure produite sur la masse de sucre mis en oeuvre lors de la multiplication des levures.

La présente invention a ainsi pour objet un procédé d'obtention de souches de levure stables tel que défini ci-dessus, comprenant une étape supplémentaire de sélection d'au moins un hybride ayant un rendement de biomasse supérieur ou égal à 90% du rendement de biomasse de la souche I-4072, de préférence supérieur ou égal à 95%, plus préférentiellement supérieur ou égal à 98%.

Cette étape supplémentaire de sélection sur la base du rendement de biomasse est effectuée avant ou après l'étape de sélection des hybrides sur le critère de stabilité et, le cas échéant, avant ou après l'étape de sélection sur la base de la performance en terme de production d'alcool.

De manière avantageuse, le rendement de biomasse des souches selon l'invention est supérieur ou égal à 90%, de préférence supérieur ou égal à 95%, plus préférentiellement supérieur ou égal à 98% du rendement de biomasse obtenu avec la souche I-4072.

Dans un mode de réalisation préféré, le procédé d'obtention de souches de levure stables comprend une présélection au niveau des ségrégeants.

Les ségrégeants obtenus après sporulation des levures sont alors sélectionnés sur la base de la stabilité et de la performance.

La présente invention a ainsi pour objet un procédé d'obtention de nouvelles souches de levure stables tel que défini ci-dessus, caractérisé en ce que l'étape de croisement de souches de levure comprend les étapes suivantes :

- sporulation de ladite souche de *Saccharomyces* instable et éventuellement de ladite autre souche de *Saccharomyces,* pour obtenir des ségrégeants,
- culture de chaque ségrégeant, pour obtenir une population R0 pour chaque ségrégeant,
- réalisation de m repiquages successifs de chaque ségrégeant, pour obtenir une population Rm de chaque ségrégeant, m étant un nombre entier supérieur ou égal à 6,
- mesure de la production d'alcool de la population R0 et celle de la production d'alcool de la population Rm de chaque ségrégeant,
- sélection des ségrégeants pour lesquels :

  ○ la production d'alcool de la population Rm est supérieure ou égale à 70% de celle de la population R0 dudit ségrégeant et
  ○ la production d'alcool de la population Rm est supérieure ou égale à 85% de celle d'une population R0 de la souche I-4072,

  pour obtenir des ségrégeants stables et performants, et
- croisement de ségrégeants stables et performants issus de ladite souche instable avec des ségrégeants stables et performants de signe opposé issus de ladite souche instable ou de ladite autre souche, pour obtenir au moins un hybride.

Les étapes de culture de chaque ségrégeant, de réalisation de m repiquage successifs et de mesure de la production d'alcool sont similaires à celles du procédé d'évaluation de la stabilité d'une souche.

Les conditions de mise en oeuvre de ces étapes sont donc les mêmes que celles décrites ci-dessus dans le cadre du procédé d'évaluation de la stabilité d'une souche.

L'étape de sélection des ségrégeants sur la base de la stabilité et de la performance en terme de production d'alcool aboutit à l'obtention de ségrégeants stables et performants, sur des critères moins sélectifs que ceux appliqués aux hybrides ou aux souches de levure.

Dans un mode de réalisation particulier du procédé d'obtention de souches de levure stables, ladite souche de levure instable est la souche I-4072.

Dans un mode de réalisation particulier du procédé d'obtention de souches de levure stables, ladite autre souche de levure est choisie parmi la souche I-4071, la souche I-4073, la souche I-4074 ou la souche I-4075.

Ladite autre souche de levure est de préférence une souche de *Saccharomyces cerevisiae.*

La présente invention a également pour objet une souche de levure susceptible d'être obtenue par le procédé d'obtention de souches tel que défini ci-dessus.

La présente invention a notamment pour objet une souche de levure obtenue par le procédé d'obtention de souches tel que défini ci-dessus.

La présente invention a également pour objet toutes les souches dérivées des souches de levure stables selon l'invention qui partagent les mêmes propriétés.

Par l'expression « souche dérivée », on désigne une souche dérivée par toute transformation quelle qu'elle soit, comme par exemple un ou plusieurs croisements et/ou une ou plusieurs mutations et/ou une ou plusieurs transformations génétiques.

Une souche dérivée par croisement peut être obtenue par croisement d'une souche selon l'invention avec la même souche, ou une autre souche selon l'invention, ou une autre souche quelconque.

Une souche dérivée par mutation peut être une souche qui a subi au moins une mutation spontanée dans son génome ou au moins une mutation induite, par exemple par mutagenèse.

La ou les mutations de la souche dérivée sont silencieuses ou non.

Par le terme « mutagenèse », on désigne à la fois la mutagenèse classique obtenue par rayonnements ou par des agents chimiques mutagènes et la mutagenèse insertionnelle par transposition ou par intégration d'un fragment d'ADN exogène.

La mutagenèse par rayonnements comprend l'utilisation de rayonnements UV, X, ou gamma.

Les agents chimiques mutagènes sont par exemple l'EMS (éthyl-méthyl sulfonate), l'EES (éthyl-éthyl sulfonate), la nitrosoguanidine, l'acide nitreux, l'aflatoxine B1, l'hydroxylamine, la 5-bromo uracile, la 2-amino purine, la proflavine, l'acridine orange.

Une souche dérivée par transformation génétique est une souche dans laquelle a été introduit un ADN exogène.

Ledit ADN exogène peut être apporté par un plasmide.

Ledit ADN exogène est de préférence intégré dans le génome de la levure.

La présente invention a ainsi pour objet une souche de *Saccharomyces cerevisiae* dérivée d'une souche de levure stable telle que définie ci-dessus, caractérisée en ce que :

- la production d'alcool d'une population Rn de ladite souche dérivée est supérieure ou égale à 95% de la production d'alcool d'une population R0 de ladite souche dérivée, et/ou

- la production d'alcool d'une population R0 de ladite souche dérivée est supérieure ou égale à 95% de la production d'alcool d'une population R0 de la souche déposée à la CNCM sous le numéro I-4072,

la population Rn étant issue de n repiquages successifs de la population R0, n étant un nombre entier supérieur ou égal à 6.

Le nombre de repiquages est, de préférence, égal à 12 et il s'agit, de préférence, de repiquages journaliers.

La présente invention a particulièrement pour objet une souche de *Saccharomyces cerevisiae* dérivée d'une souche telle que définie ci-dessus, caractérisée en ce que :

- la production d'alcool d'une population Rn de ladite souche dérivée est supérieure ou égale à 95% de la production d'alcool d'une population R0 de ladite souche dérivée, de préférence supérieure ou égale à 97%, plus préférentiellement supérieure ou égale à 99%, et

- la production d'alcool d'une population R0 de ladite souche dérivée est supérieure ou égale à 95% de la production d'alcool d'une population R0 de la souche déposée à la CNCM sous le numéro I-4072, de préférence supérieure ou égale à 97%, plus préférentiellement supérieure ou égale à 99%,

la population Rn étant issue de n repiquages successifs de la population R0, n étant un nombre entier supérieur ou égal à 6.

L'invention a également pour objet un procédé de transformation d'une souche de levure stable, pour obtenir une souche dérivée telle que définie ci-dessus, ledit procédé de transformation comprenant une étape de transformation de ladite souche par au moins un croisement et/ou au moins une mutation et/ou au moins une transformation génétique.

La présente invention a également pour objet une levure obtenue par culture d'une souche de levure telle que définie ci-dessus.

Les levures sont produites à partir de souches de levure stables selon l'invention, notamment comme décrit dans le livre de référence « Yeast Technology », 2ème édition, 1991, G. Reed et T.W. Nagodawithana, publié par Van Nostrand Reinhold, ISBN 0-442-31892-8.

La multiplication des levures, à l'échelle industrielle, comprend en général au moins les deux premières étapes et la dernière étape de l'ensemble des étapes suivantes :

- multiplication d'une souche de levure en plusieurs stades, d'abord en semi-anaérobiose, puis en aérobiose,
- séparation par centrifugation de la levure ainsi produite de son milieu de culture, pour obtenir une crème de levure liquide contenant environ entre 12 et 25% de matière sèche, voire une quantité plus élevée de matière sèche si la crème de levure est mélangée avec des produits osmolytes,
- filtration de la crème de levure liquide ainsi obtenue, en général sur un filtre rotatif sous vide, pour obtenir une levure fraîche déshydratée contenant de 26% à 35% de matière sèche,
- malaxage de ladite levure fraîche déshydratée, pour obtenir une masse homogène,
- extrusion de la levure ainsi obtenue, pour obtenir

  ◦ une levure pressée sous forme de pains de levure fraîche ou de levure fraîche émiettée, contenant environ 30% de matière sèche, ou
  ◦ une levure sous forme de particules, en général de granules, si la levure est destinée à être séchée,

- éventuellement, séchage de manière ménagée, dans un courant d'air chaud, par exemple par fluidisation, des particules de levures obtenues par extrusion pour obtenir de la levure sèche,
- emballage.

L'étape de séchage est de préférence un séchage rapide ménageant en présence d'un émulsifiant.

Parmi les émulsifiants pouvant être utilisés lors de l'étape de séchage, on peut choisir le monostéarate de sorbitan, utilisé par exemple à une concentration d'environ 1,0% (en poids sur le poids de levure sèche).

Les levures selon l'invention sont des levures de qualité fiable et, de préférence, performantes en terme de production d'alcool.

Les levures selon l'invention peuvent être utilisées sous toute forme possible.

Par exemple, la présente invention a pour objet une levure telle que définie ci-dessus, caractérisée en ce qu'elle est sous forme de crème de levure, de levure pressée, de levure sèche ou de levure surgelée.

Les levures fraîches sont caractérisées par une teneur élevée en eau en comparaison aux levures sèches. Les levures fraîches englobent les crèmes de levure et les levures pressées.

Les crèmes de levure, appelées également « levures liquides », sont des suspensions aqueuses de cellules de levure présentant une viscosité de type crème.

Par crème de levure, on comprend une suspension liquide, typiquement une suspension aqueuse, de cellules vivantes de levure, ladite suspension ayant une teneur en matière sèche d'au moins 12% en masse, généralement comprise d'environ 12 à environ 50% en masse (définition étendue de crème de levure).

De préférence, la crème de levure répond à la définition au sens strict, c'est-à-dire qu'elle présente une teneur en matière sèche comprise d'environ 12 à environ 25% en masse, de préférence d'environ 14 à environ 22% en masse.

Parmi les levures pressées, on distingue les levures pressées en bloc compact, appelées également « pains de levure » qui sont caractérisées par une teneur en matière sèche comprise d'environ 26% à environ 35%, et les levures pressées en granules qui sont caractérisées par une teneur en eau comprise d'environ 21% à environ 35%.

Les levures sèches sont caractérisées par une teneur en matière sèche supérieure à environ 92%.

Les levures surgelées sont caractérisées par une teneur en matière sèche comprise d'environ 74% à environ 80%.

La levure selon l'invention présente donc les caractéristiques recherchées pour une levure destinée à la production d'alcool, à savoir une qualité fiable et une production d'alcool performante.

La présente invention a ainsi également pour objet l'utilisation d'une levure telle que définie ci-dessus pour produire de l'alcool.

Les conditions de la fermentation alcoolique dépendent particulièrement du type d'application recherchée, par exemple selon qu'il s'agit d'une fermentation de type brasserie, vinification ou distillerie.

Les conditions de fermentation alcoolique sont facilement déterminables par l'homme du métier.

A titre d'exemple, on peut se référer aux conditions de fermentation alcoolique décrites dans le livre de référence « Yeast Technology », 2ème édition, 1991, G. Reed et T.W. Nagodawithana, publié par Van Nostrand Reinhold, ISBN 0-442-31892-8.

Les levures selon l'invention sont particulièrement intéressantes dans les applications suivantes :

- l'alcool dit « de bouche », destiné à la fabrication de boissons alcoolisées, et/ou
- l'alcool industriel, destiné par exemple aux biocarburants ou aux industries chimiques.

La présente invention va maintenant être illustrée à l'aide des exemples suivants qui sont donnés à titre d'illustration et ne sont nullement limitatifs.

Les exemples décrivent en particulier un procédé d'obtention de souches de levure stables selon l'invention et leurs caractéristiques, ainsi que les caractéristiques de levures obtenues à partir de ces souches.

EXEMPLE 1: SELECTION DE SOUCHES DE LEVURE STABLES ET LEURS CARACTERISTIQUES

**Matériel et Méthodes**

1. Milieux et produits

La composition des milieux de culture et la nature des enzymes utilisées sont indiquées ci-dessous.

| Milieu 1 | | Milieu 2 | |
|---|---|---|---|
| Extrait de levure | 3g | Acétate de sodium | 6.5g |
| Extrait de malt | 3g | Agar | 15g |
| Peptone | 5g | pH : 6.5-7 | |
| Glucose | 10g | Eau qsp 1L | |
| Agar | 20g | stérilisation : 20 minutes à 121°C | |
| pH : 6.2 +/- 0.2 | | | |
| Eau qsp 1L | | | |
| stérilisation : 20 minutes à 121 °C | | | |

(suite)

| Milieu 3 | | Milieu 4 | |
|---|---|---|---|
| Mélasse de canne | 5g | Saccharose | 100g |
| (NH4)2HPO4 | 500mg | Extrait de levure | 20 g |
| Agar | 30g | MgSO4 | 1 g |
| pH ajusté à 5.2-5.3 avec H2SO4 10% | | KH2PO4 | 1 g |
| Eau qsp 1L | | pH ajusté à 4.7 par de l'acide lactique | |
| stérilisation : 40 minutes à 121 °C | | Eau qsp 1L | |
| | | stérilisation : 30 minutes à 121°C | |

| Milieu 5 | | Enzymes : | |
|---|---|---|---|
| Saccharose | 240 g | Zymolyase 100000 à 20mg /mL | |
| (NH4)2 HP04 | 4.7 g | | |
| KCl | 0.8 g | | |
| Tween 80 | 0.5 ml | | |
| Tampon citrate | 0,2 M | | |
| Vitamine B1 (Thiamine) | 4mg | | |
| Vitamine B6 (Pyridoxine) | 4mg | | |
| Acide Nicotinique | 40mg | | |
| Biotine0.01mg | | | |
| pH : 4.2 | | | |
| Eau qsp 1L | | | |

Le milieu 1 est un milieu de culture basique gélosé contenant les éléments nécessaires à la croissance des souches de *Saccharomyces cerevisiae* selon l'invention.

Le milieu 2 est un milieu gélosé approprié pour la sporulation des levures.

Le milieu 3 est un milieu de culture gélosé approprié pour la croissance de levures. Le milieu 3 contient de la mélasse, le substrat généralement utilisé pour la multiplication industrielle des levures.

Le milieu 4 est un milieu de culture liquide standard approprié pour la multiplication des levures.

Le milieu 5 est un milieu de culture liquide approprié pour réaliser une fermentation alcoolique.

La zymolase est une enzyme utilisée après la sporulation, afin de digérer partiellement la paroi des asques et faciliter leur dissection.

2. Isolement des ségrégeants

Etape 1 : Croissance de la souche sur du milieu 1

Une öse de la souche de départ (conservée à -80°C) est ensemencée en surface de la gélose d'une boîte de Pétri. La boîte de Pétri est ensuite mise en incubation pendant 24 heures à 30°C.

Etape 2 : Sporulation sur du milieu 2

Une öse de la culture précédente est ensemencée en surface de la gélose d'une boîte de Pétri. La boîte de Pétri est mise en incubation pendant 96 heures à 25°C. La biomasse obtenue en surface de la boîte est ensuite récoltée dans 500µL d'eau stérile. A 100µL de cette suspension sont ajoutés 25µl de zymolyase. La suspension est mise en incubation pendant 30 minutes à 30°C, avant d'être étalée sur boîte gélosée de milieu 1. De préférence, la dissection des tétrades est effectuée 20 minutes plus tard.

Etape 3 : Dissection des tétrades

Les tétrades sont disséquées au micro-manipulateur SINGER, puis la boîte de pétri est mise en incubation pendant 24 heures à 30°C.

Etape 4 : Conservation des ségrégeants

Les ségrégeants sont ensuite conservés à -80°C dans du milieu 1 contenant 20% de glycérol.

3. Repiquages successifs et nombre de générations

Etape 1 : Croissance sur du milieu 1

Une öse de la souche à tester (conservée à -80°C) est ensemencée en surface de la gélose d'une boîte de Pétri contenant du milieu 1. La boîte de Pétri est ensuite mise en incubation pendant 24 heures à 30°C.

Etape 2 : Repiquages successifs sur du milieu 3

La souche à tester est repiquée en surface d'une boîte de pétri contenant du milieu 3 à l'aide d'une pointe de cure dent. La boîte de pétri est ensuite mise en incubation pendant 20 à 24 heures à 30°C.
Les étapes suivantes sont alors répétées 11 fois :

- repiquage de la souche à tester en surface d'une boîte de pétri contenant du milieu 3 à l'aide d'une pointe de cure dent,
- mise en incubation de la boite de Pétri, pendant 20 à 24 heures à 30°C.

Variante de l'étape 2

De manière alternative, les repiquages successifs sur le milieu 3 peuvent être réalisés selon le protocole suivant.
La biomasse obtenue à l'étape 1 est récupérée et diluée dans de l'eau distillée, afin d'obtenir une suspension de levures à 0,5 mg/ml. Une boîte de Pétri contenant du milieu 3 est alors ensemencée en surface avec 100$\mu$L de cette suspension.
La boîte de Pétri est mise en incubation pendant 20 à 24 heures à 30°C.
Les étapes suivantes sont alors répétées 11 fois :

- la biomasse obtenue est récoltée dans 2 x 2,5mL d'eau stérile,
- des dilutions sont effectuées afin d'obtenir une suspension de levures à 0,5 mg/ml,
- à partir de cette suspension, une boîte de Pétri contenant du milieu 3 est à nouveau ensemencée en surface avec 100$\mu$L de ladite suspension, et
- la boîte de Pétri est mise en incubation pendant 20-24 heures à 30°C.

Etape 3 : Calcul du nombre de générations obtenues à l'étape 2

Soit $n_g$, le nombre de générations et $X_0$, la biomasse ensemencée, la biomasse $X_n$ obtenue à l'issue de n repiquages est donnée par la formule suivante :

$$X_n = 2^{ng} X_o$$

Le nombre de générations est donc calculé en appliquant la formule suivante :

$$n_g = (\log X_t - \log X_0) / \log 2, \text{ soit } n_g = 3,3 \log (X_n / X_o)$$

La biomasse peut être exprimée en nombre de cellules ou en poids de matière sèche.
S'agissant du nombre de cellules, les cellules de levure prélevées sur une pointe de cure dent ou obtenues à l'issue de l'incubation sur la boîte de Pétri sont mises en suspension dans 1 ml d'eau. Le nombre de cellules est ensuite obtenu par mesure au cytomètre en flux.
Environ 5 générations sont obtenues entre chaque repiquage, ce qui correspond en moyenne à 60 générations sur 12 repiquages successifs.

Variante de l'étape 3

Le nombre de générations obtenu à l'issue de la variante de l'étape 2 est déterminé de la manière suivante.

Le poids de matière sèche ($X_o$) de ladite suspension de levures est mesuré, ainsi que le poids de matière sèche ($X_t$) de la suspension de levures récoltée après 20 à 24 heures de croissance. Le poids de matière sèche est mesuré après déshydratation dans une étuve à environ 105°C. Le nombre de générations est calculé en appliquant la formule suivante :

$$n_g = 3,3 \log (X_n/ X_o)$$

3 à 4 générations sont obtenues entre chaque repiquage, ce qui correspond à environ 36 à environ 48 générations sur 12 repiquages successifs.

4. Test de fermentation alcoolique par mesure de l'éthanol produit

Etape 1 : Culture sur le milieu 1

Une öse de la souche à tester (conservée à -80°C) est ensemencée en surface d'une boite de Pétri contenant du milieu 1. La boîte de Pétri est mise en incubation pendant 24 heures à 30°C.

Etape 2 : Pré-culture en milieu liquide en fiole de 50mL

Une öse de la culture précédente est ensemencée dans une fiole de 50 mL contenant 20 mL de milieu 4. La fiole est mise en incubation pendant 24 heures à 26°C.

Etape 3 : Culture en milieu liquide en fiole de 250mL

6mL de la culture obtenue à l'étape précédente sont ensemencés dans une fiole de 250 mL contenant 150mL de milieu 4. La fiole est mise en incubation pendant 20 heures à 26°C. La culture obtenue est centrifugée à 4500 rpm pendant 3 minutes, le surnageant est éliminé et le culot est remis en suspension dans 100mL d'eau distillée stérile (lavage). La suspension est à nouveau centrifugée à 4500 rpm pendant 3 minutes et le culot est remis en suspension dans 20 mL d'eau distillée stérile.

Le poids de matière sèche est mesuré.

Etape 4 : Fermentation alcoolique en ballon de 4000mL

1g de matière sèche levure de la suspension obtenue lors de l'étape 3 est ensemencé dans un ballon de 4000mL contenant 1000mL de milieu 5.

Le ballon est mis en incubation pendant 72 heures à 35°C sous agitation 80rpm.

10mL du moût de fermentation sont prélevés à t=0, t=7 heures, t=24 heures, t=48heures et t=72heures.

Le moût de fermentation prélevé est filtré (filtre à 0.2µ) et la solution de fermentation ainsi obtenue est conservée à -20°C.

La concentration en sucre, éthanol et glycérol présents dans la solution de fermentation est ensuite mesurée par HPLC (colonne HPX87H).

**Résultats**

1. Sélection des ségrégeants

Des ségrégeants ont été isolés des souches déposées le 4 septembre 2008 à la CNCM sous les numéros I-4071 et I-4072, comme indiqué dans la section « Matériel et Méthodes ».

L'étape de sporulation aboutit à la formation d'asques contenant 4 spores haploïdes qui sont disséquées.

Chaque spore haploïde constitue un ségrégeant.

Les ségrégeants issus des deux souches I-4071 et I-4072 sont évalués en terme de quantité d'alcool produit et de stabilité.

Les résultats de 5 tétrades issues de la souche I-4072 (numérotées de 1 à 5) sont présentés dans les tableaux 1. a) à e).

Les résultats de 6 tétrades issues de la souche I-4071 (numérotées de 6 à 11) sont présentés dans les tableaux 2. a) à f).

Les valeurs données dans les tableaux correspondent à la moyenne obtenue sur au moins trois essais indépendants.

La ligne (1) indique la production d'alcool de la population R0 du ségrégeant, exprimée en % de celle de la population R0 de la souche I-4072, et renseigne donc sur la performance du ségrégeant.

La ligne (2) indique la production d'alcool de la population R12 du ségrégeant, exprimée en % de celle de la population R0 dudit ségrégeant, et renseigne donc sur la stabilité du ségrégeant.

Tableau 1. a)

| Tétrade 1 | 1a | **1b** | 1c | **1d** |
|---|---|---|---|---|
| signe | a | **a** | $\alpha$ | $\alpha$ |
| (1) | 77 | **97** | 64 | **101** |
| (2) | 103 | **88** | 108 | **77** |

Tableau 1. b)

| Tétrade 2 | 2a | 2b | 2c | **2d** |
|---|---|---|---|---|
| signe | a | a | $\alpha$ | $\alpha$ |
| (1) | 100 | 65 | 73 | **100** |
| (2) | 68 | 96 | 101 | **80** |

Tableau 1. c)

| Tétrade 3 | **3a** | 3b | 3c | 3d |
|---|---|---|---|---|
| signe | **a** | a | $\alpha$ | $\alpha$ |
| (1) | **98** | 82 | 97 | 66 |
| (2) | **82** | 100 | 60 | 99 |

Tableau 1. d)

| Tétrade 4 | 4a | **4b** | **4c** | 4d |
|---|---|---|---|---|
| signe | a | **a** | $\alpha$ | $\alpha$ |
| (1) | 75 | **94** | **98** | 71 |
| (2) | 98 | **100** | **88** | 100 |

Tableau 1. e)

| Tétrade 5 | **5a** | 5b | 5c | 5d |
|---|---|---|---|---|
| signe | **a** | a | $\alpha$ | $\alpha$ |
| (1) | **99** | 97 | 65 | 68 |
| (2) | **109** | 67 | 121 | 103 |

Tableau 2. a)

| Tétrade 6 | **6a** | **6b** | 6c | 6d |
|---|---|---|---|---|
| signe | **a** | **a** | α | α |
| (1) | **99** | **98** | 56 | 81 |
| (2) | **93** | **94** | 99 | 98 |

Tableau 2. b)

| Tétrade 7 | 7a | **7b** | **7c** | 7d |
|---|---|---|---|---|
| signe | a | **a** | α | α |
| (1) | 63 | **97** | **102** | 52 |
| (2) | 103 | **97** | **97** | 154 |

Tableau 2. c)

| Tétrade 8 | 8a | **8b** | 8c | **8d** |
|---|---|---|---|---|
| signe | a | **a** | α | α |
| (1) | 53 | **98** | 58 | **100** |
| (2) | 109 | **99** | 100 | **76** |

Tableau 2. d)

| Tétrade 9 | 9a | **9b** | **9c** | 9d |
|---|---|---|---|---|
| signe | a | **a** | α | α |
| (1) | 54 | **101** | **101** | 49 |
| (2) | 126 | **95** | **97** | 133 |

Tableau 2. e)

| Tétrade 10 | 10a | **10b** | **10c** | 10d |
|---|---|---|---|---|
| signe | a | **a** | α | α |
| (1) | 49 | **96** | **100** | 56 |
| (2) | 104 | **95** | **78** | 107 |

Tableau 2. f)

| Tétrade 11 | 11a | 11b | 11c | 11d |
|---|---|---|---|---|
| signe | a | a | α | α |
| (1) | 52 | **99** | 52 | **96** |
| (2) | 146 | **88** | 125 | **99** |

En caractères gras figurent les tétrades qui répondent aux critères de sélection appliqués aux ségrégeants : production d'alcool supérieure ou égale à 90% pour la ligne (1) et supérieure ou égale à 75% pour la ligne (2).

2. <u>Croisement et sélection des hybrides</u>

Parmi les ségrégeants sélectionnés, les ségrégeants de signe $\alpha$ sont croisés avec ceux de signe a, pour obtenir des hybrides.

Les hybrides sont ensuite sélectionnés sur la base des critères suivants :

- stabilité : la production d'alcool de la population Rn est supérieure ou égale à 97% de celle de la population R0, et
- performance : la production d'alcool de la population R0 est supérieure ou égale à 95% de celle de la population R0 de la souche I-4072.

Les tableaux 3 et 4 indiquent les résultats obtenus avec les hybrides correspondant aux souches selon l'invention :

- souches I-4073 et I-4074 : hybrides issus du croisement du ségrégeant 1d avec le ségrégeant 7b, et
- souche I-4075 : hybride issu de croisement du ségrégeant 1d avec le ségrégeant 5a.

Les résultats obtenus avec la souche I-4072 sont également indiqués.

Les tableaux 3.a) et 3.b) indiquent les résultats obtenus dans le test de fermentation alcoolique par mesure de l'éthanol produit, respectivement à 48h et 72h.

La ligne (1) indique la production d'alcool de la population R0 de la souche exprimée en pourcentage de celle de la population R0 de la souche I-4072, et renseigne donc sur la performance de la souche.

La ligne (2) indique la production d'alcool de la population R12 de la souche exprimée en pourcentage de celle de la population R0 de ladite souche, et renseigne donc sur la stabilité de la souche.

Les valeurs données dans les tableaux correspondent à la moyenne obtenue sur au moins trois essais.

Tableau 3. a)

| Souche | (1) | (2) |
|--------|-------|------|
| I-4072 | 100,0 | 48,9 |
| I-4073 | 99,9 | 99,1 |
| I-4074 | 100,7 | 98,5 |
| I-4075 | 99,8 | 97,8 |

Tableau 3. b)

| Souche | (1) | (2) |
|--------|-------|------|
| I-4072 | 100,0 | 46,9 |
| I-4073 | 99,7 | 98,3 |
| I-4074 | 99,1 | 99,2 |
| I-4075 | 99,9 | 99,0 |

Les hybrides sélectionnés sont très performants en terme de production d'alcool, puisque la quantité d'alcool produit est supérieure à 99% de celle obtenue avec la souche de référence I-4072 (tableaux 3.a) et 3.b)).

Les hybrides montrent également une très forte stabilité : la production d'alcool entre la population R0 et R12 a diminué de moins de 3% pour les hybrides I-4073, I-4074 et I-4075.

En comparaison, la production d'alcool de la souche I-4072 a chuté de plus de 50% (tableaux 3.a) et 3.b)).

Pour rappel, les résultats de la population R12 correspondent à une levure ayant été multipliée sur environ 60 générations, ce qui est supérieur ou égal au nombre de générations moyen obtenu à l'issue d'une multiplication industrielle de levures.

EXEMPLE 2: CARACTERISTIQUES DES LEVURES SELON L'INVENTION

**Matériel et Méthodes**

**1. Production à l'échelle pilote**

Les souches de levure I-4073, I-4074 et I-4075 sont multipliées dans des fermenteurs de 20 litres, en mode semi-continu, comme décrit dans le livre de référence « Yeast Technology », 2ème édition, 1991, G. Reed et T.W. Nagodawithana, publié par Van Nostrand Reinhold, ISBN 0-442-31892-8.

Les levures obtenues à partir de chaque souche sont ensuite testées en terme de stabilité, de performance, et de rendement de biomasse.

L'évaluation de la stabilité des levures est effectuée selon le test d'évaluation de stabilité utilisé pour les souches de levures, sur 12 repiquages successifs journaliers.

La performance est évaluée en mesurant la quantité maximale d'alcool produit.

Le rendement de biomasse est obtenu en calculant la masse de levure produite sur la masse de sucre mis en oeuvre lors de la multiplication des levures.

**2. Production industrielle**

La souche de levure I-4074 est multipliée à l'échelle industrielle.

L'évaluation de la stabilité des levures obtenues à l'issue des multiplications à l'échelle industrielle est effectuée selon le test d'évaluation de stabilité utilisé pour les souches de levures, sur 12 repiquages successifs journaliers.

La performance est évaluée en mesurant la quantité maximale d'alcool produit.

**Résultats**

Les résultats obtenus après multiplications des souches de levure à l'échelle pilote montrent que les levures issues de chacune des trois souches de levure I-4073, I-4074 et I-4075 sont stables, donc de qualité fiable, performantes en terme de quantité d'alcool produit, et possèdent un bon rendement de biomasse.

De plus, après multiplication industrielle, les levures obtenues à partir de la souche de levure I-4074 sont stables, donc de qualité fiable. De plus, ces levures sont également performantes en terme de quantité d'alcool produit et possèdent un bon rendement de biomasse.

**Revendications**

**1.** Souche de *Saccharomyces cerevisiae* stable choisie parmi la souche déposée à la CNCM sous le numéro I-4073, la souche déposée à la CNCM sous le numéro I-4074 ou la souche déposée à la CNCM sous le numéro I-4075.

**2.** Procédé comprenant les étapes de :

- croisement d'une souche de *Saccharomyces* instable avec elle-même ou avec une autre souche de *Saccharomyces,* pour obtenir au moins un hybride,
- culture d'au moins un hybride, pour obtenir une population de levure R0,
- réalisation de n repiquages successifs de la population de levure R0, n étant un nombre entier supérieur ou égal à 6, pour obtenir une population de levures Rn,
- mesure de la production d'alcool de ladite population R0 et celle de la production d'alcool de ladite population Rn,
- sélection d'au moins un hybride pour lequel la production d'alcool de la population Rn est supérieure ou égale à 95% de celle de la population R0, pour obtenir des souches de levure stables, où
- la souche de levure stable est une souche de levure qui donne des levures dont la production d'alcool est stable au fil des générations ; et
- la souche de levure instable est une souche de levure qui donne des levures dont la production d'alcool n'est pas stable au fil des générations.

**3.** Procédé selon la revendication 2, comprenant une étape supplémentaire de sélection d'au moins un hybride pour lequel la production d'alcool de la population R0 est supérieure ou égale à 95% de celle d'une population RO de la souche I-4072.

**4.** Procédé selon la revendication 2 ou 3, comprenant une étape supplémentaire de sélection d'au moins un hybride ayant un rendement de biomasse supérieur ou égal à 90% du rendement de biomasse de la souche I-4072.

**5.** Procédé selon la revendication 2, dans lequel l'étape de croisement comprend les étapes suivantes :

- sporulation de ladite souche de *Saccharomyces* instable et éventuellement de ladite autre souche de *Saccharomyces*, pour obtenir des ségrégeants,
- culture de chaque ségrégeant, pour obtenir une population R0 pour chaque ségrégeant,
- réalisation de m repiquages successifs de chaque ségrégeant, pour obtenir une population Rm de chaque ségrégeant, m étant un nombre entier supérieur ou égal à 6,
- mesure de la production d'alcool de la population R0 et celle de la production d'alcool de la population Rm de chaque ségrégeant,
- sélection des ségrégeants pour lesquels :

o la production d'alcool de la population Rm est supérieure ou égale à 70% de celle de la population R0 dudit ségrégeant, et
o la production d'alcool de la population R0 est supérieure ou égale à 85% de celle d'une population R0 de la souche I-4072,

pour obtenir des ségrégeants stables et performants, et
- croisement de ségrégeants stables et performants issus de ladite souche instable avec des ségrégeants stables et performants de signe opposé issus de ladite souche instable ou de ladite autre souche, pour obtenir au moins un hybride.

**6.** Procédé selon l'une des revendications 2 à 5, dans lequel ladite souche de levure instable est la souche I-4072.

**7.** Procédé selon l'une des revendications 2 à 6, dans lequel ladite autre souche est choisie parmi la souche I-4071, la souche I-4073, la souche I-4074 ou la souche I- 4075.

**8.** Souche de *Saccharomyces cerevisiae* dérivée d'une souche selon la revendication 1, **caractérisée en ce que** :

- la production d'alcool d'une population Rn de ladite souche dérivée est supérieure ou égale à 95% de la production d'alcool d'une population R0 de ladite souche dérivée, et/ou
- la production d'alcool d'une population R0 de ladite souche dérivée est supérieure ou égale à 95% de la production d'alcool d'une population R0 de la souche déposée à la CNCM sous le numéro I-4072,

la population Rn étant issue de n repiquages successifs de la population R0, n étant un nombre entier supérieur ou égal à 6.

**9.** Procédé d'évaluation de la stabilité d'une souche de levure, comprenant les étapes de :

- culture de ladite souche, pour obtenir une population de levure R0,
- réalisation de n repiquages successifs de la population de levure R0, n étant un nombre entier supérieur ou égal à 6, pour obtenir une population de levures Rn,
- mesure de la production d'alcool de ladite population R0 et celle de la production d'alcool de ladite population Rn,
- qualification de ladite souche de " stable " si la production d'alcool de la population Rn est supérieure ou égale à 95% de la production d'alcool de la population R0.

**10.** Levure obtenue par culture d'une souche de levure selon la revendication 1.

**11.** Levure selon la revendication 10, **caractérisée en ce que** sa production d'alcool est supérieure ou égale à 95% de la production d'alcool d'une levure issue de la souche I- 4072.

**12.** Levure selon la revendication 10 ou 11, **caractérisée en ce qu'**elle est sous forme de crème de levure, de levure pressée, de levure sèche ou de levure surgelée.

**13.** Utilisation d'une levure selon l'une des revendications 10 à 12, pour produire de l'alcool.

**Patentansprüche**

1.  Stabiler *Saccharomyces cerevisiae* Stamm, ausgewählt aus dem bei der CNCM unter der Nummer I-4073 hinterlegten Stamm, dem bei der CNCM unter der Nummer I-4074 hinterlegten Stamm oder dem bei der CNCM unter der Nummer I-4075 hinterlegten Stamm.

2.  Verfahren, das die folgenden Schritte umfasst:

    - Kreuzen von einem instabilen *Saccharomyces* Stamm mit sich selbst oder mit einem anderen *Saccharomyces* Stamm, um mindestens einen Hybrid zu erhalten,
    - Kultivieren von mindestens einem Hybrid, um eine Hefepopulation R0 zu erhalten,
    - Anlegen von n aufeinanderfolgenden Subkulturen der Hefepopulation R0, wobei n eine ganze Zahl ist, die größer oder gleich 6 ist, um eine Hefepopulation Rn zu erhalten,
    - Messen der Alkoholherstellung von besagter Population R0 und das der Alkoholherstellung von besagter Population Rn,
    - Auswählen von mindestens einem Hybrid, für den die Alkoholherstellung der Population Rn mehr als oder gleich 95% von der der Population R0 beträgt, um stabile Hefestämme zu erhalten, wobei
    - der stabile Hefestamm ein Hefestamm ist, der Hefen ergibt, deren Alkoholherstellung über Generationen stabil ist; und
    - der instabile Hefestamm ein Hefestamm ist, der Hefen ergibt, deren Alkoholherstellung nicht über Generationen stabil ist.

3.  Verfahren gemäß Anspruch 2, umfassend einen zusätzlichen Schritt, bei dem mindestens ein Hybrid ausgewählt wird, für den die Alkoholherstellung der Population R0 mehr als oder gleich 95% von der einer Population R0 des Stamms I-4072 beträgt.

4.  Verfahren gemäß Anspruch 2 oder 3, umfassend einen zusätzlichen Schritt, bei dem mindestens ein Hybrid ausgewählt wird, dessen Biomassenertrag mehr als oder gleich 90% des Biomassenertrags des Stamms I-4072 beträgt.

5.  Verfahren gemäß Anspruch 2, bei dem der Kreuzungsschritt die folgenden Schritte umfasst:

    - Sporulation von besagtem instabilen *Saccharomyces* Stamm und gegebenenfalls von besagtem anderen *Saccharomyces* Stamm um Segregate zu erhalten,
    - Kultivieren von jedem Segregat, um eine Population R0 für jedes Segregat zu erhalten,
    - Anlegen von m aufeinanderfolgenden Subkulturen von jedem Segregat, um eine Population Rm von jedem Segregat zu erhalten, wobei m eine ganze Zahl ist, die größer oder gleich 6 ist,
    - Messen der Alkoholherstellung der Population R0 und das der Alkoholherstellung der Population Rm von jedem Segregat,
    - Auswählen von Segregaten für die:

        ◦ die Alkoholherstellung der Population Rm mehr als oder gleich 70% von der der Population R0 von besagtem Segregat beträgt, und
        ◦ die Alkoholherstellung der Population R0 mehr als oder gleich 85% von der einer Population R0 des Stamms I-4072 beträgt,

    um stabile und leistungsfähige Segregate zu erhalten, und
    - Kreuzen von aus besagtem instabilen Stamm gewonnenen stabilen und leistungsfähigen Segregaten mit aus besagtem instabilen Stamm oder besagtem anderen Stamm gewonnenen stabilen und leistungsfähigen Segregaten mit entgegengesetztem Vorzeichen, um mindestens einen Hybrid zu erhalten.

6.  Verfahren gemäß einem der Ansprüche 2 bis 5, bei dem besagter instabiler Hefestamm der Stamm I-4072 ist.

7.  Verfahren gemäß einem der Ansprüche 2 bis 6, bei dem besagter anderer Stamm ausgewählt wird aus dem Stamm I-4071, dem Stamm I-4073, dem Stamm I-4074 oder dem Stamm I-4075.

8.  *Saccharomyces cerevisiae* Stamm, abgeleitet von einem Stamm gemäß Anspruch 1, **dadurch gekennzeichnet, dass**:

- die Alkoholherstellung einer Population Rn von besagtem abgeleiteten Stamm mehr als oder gleich 95% von der Alkoholherstellung einer Population R0 von besagtem abgeleiteten Stamm beträgt, und/oder
- die Alkoholherstellung einer Population R0 von besagtem abgeleiteten Stamm mehr als oder gleich 95% von der Alkoholherstellung einer Population R0 des bei der CNCM unter der Nummer I-4072 hinterlegten Stamms beträgt,

wobei die Population Rn aus n aufeinanderfolgenden Subkulturen der Population R0 gewonnen wird, wobei n eine ganze Zahl ist, die größer oder gleich 6 ist.

9. Verfahren zur Evaluierung der Stabilität eines Hefestamms, wobei das Verfahren die folgenden Schritte umfasst:

- Kultivieren von besagtem Stamm, um eine Hefepopulation R0 zu erhalten,
- Anlegen von n aufeinanderfolgenden Subkulturen der Hefepopulation R0, wobei n eine ganze Zahl ist, die größer oder gleich 6 ist, um eine Hefepopulation Rn zu erhalten,
- Messen der Alkoholherstellung von besagter Population R0 und das der Alkoholherstellung von besagter Population Rn,
- Einstufung von besagtem Stamm als "stabil" falls die Alkoholherstellung der Population Rn mehr als oder gleich 95% von der Alkoholherstellung der Population R0 beträgt.

10. Hefe, die durch Kultivieren eines Hefestamms gemäß Anspruch 1 erhalten wurde.

11. Hefe gemäß Anspruch 10, **dadurch gekennzeichnet, dass** ihre Alkoholherstellung mehr als oder gleich 95% von der Alkoholherstellung einer aus dem Stamm I-4072 gewonnenen Hefe beträgt.

12. Hefe gemäß Anspruch 10 oder 11, **dadurch gekennzeichnet, dass** sie in Form einer Hefecreme ("crème de levure"), Presshefe, Trockenhefe oder tiefgefrorenen Hefe vorliegt.

13. Verwendung einer Hefe gemäß einem der Ansprüche 10 bis 12, zur Herstellung von Alkohol.

**Claims**

1. A stable *Saccharomyces cerevisiae* strain chosen from the strain deposited with the CNCM under number I-4073, the strain deposited with the CNCM under number I-4074 or the strain deposited with the CNCM under number I-4075.

2. A method comprising the steps of:

- crossing an unstable *Saccharomyces* strain with itself or with another *Saccharomyces* strain, so as to obtain at least one hybrid,
- culturing at least one hybrid, so as to obtain a yeast population R0,
- carrying out n successive subcultures of the yeast population R0, n being an integer greater than or equal to 6, so as to obtain a yeast population Rn,
- measuring the alcohol production of said population R0 and measuring the alcohol production of said population Rn,
- selecting at least one hybrid for which the alcohol production of the population Rn is greater than or equal to 95% of that of the population R0, so as to obtain stable yeast strains, where
- the stable yeast strain is a yeast strain which gives yeasts from which alcohol production is stable through generations; and
- the unstable yeast strain is a yeast strain which gives yeasts from which alcohol production is not stable through generations.

3. The method as claimed in claim 2, comprising an additional step of selecting at least one hybrid for which the alcohol production of the population R0 is greater than or equal to 95% of that of a population R0 of the I-4072 strain.

4. The method as claimed in claim 2 or 3, comprising an additional step of selecting at least one hybrid which has a biomass yield greater than or equal to 90% of the biomass yield of the I-4072 strain.

5. The method as claimed in claim 2, in which the crossing step comprises the following steps:

- sporulating said unstable *Saccharomyces* strain and, optionally, said other *Saccharomyces* strain, so as to obtain segregants,
- culturing said segregant, so as to obtain a population R0 for each segregant,
- carrying out m successive subcultures of each segregant, so as to obtain a population Rm of each segregant, m being an integer greater than or equal to 6,
- measuring the alcohol production of the population R0 and measuring the alcohol production of the population Rm of each segregant,
- selecting the segregants for which:

  ○ the alcohol production of the population Rm is greater than or equal to 70% of that of the population R0 of said segregant, and
  ○ the alcohol production of the population R0 is greater than or equal to 85% of that of a population R0 of the I-4072 strain,

  so as to obtain effective, stable segregants, and
- crossing effective, stable segregants stemming from said unstable strain with effective, stable segregants of opposite sign stemming from said unstable strain or from said other strain, so as to obtain at least one hybrid.

6. The method as claimed in one of claims 2 to 5, in which said unstable yeast strain is the I-4072 strain.

7. The method as claimed in one of claims 2 to 6, in which said other strain is chosen from the I-4071 strain, the I-4073 strain, the I-4074 strain or the I-4075 strain.

8. A *Saccharomyces cerevisiae* strain derived from a strain as claimed in claim 1, **characterized in that**:

   - the alcohol production of a population Rn of said derived strain is greater than or equal to 95% of the alcohol production of a population R0 of said derived strain, and/or
   - the alcohol production of a population R0 of said derived strain is greater than or equal to 95% of the alcohol production of a population R0 of the strain deposited with the CNCM under number I-4072,

   the population Rn stemming from n successive subcultures of the population R0, n being an integer greater than or equal to 6.

9. A method for evaluating the stability of a yeast strain, comprising the steps of

   - culturing said strain, so as to obtain a yeast population R0,
   - carrying out n successive subcultures of the yeast population R0, n being an integer greater than or equal to 6, so as to obtain a yeast population Rn,
   - measuring the alcohol production of said population R0 and measuring the alcohol production of said population Rn,
   - describing said strain as "stable" if the alcohol production of the population Rn is greater than or equal to 95% of the alcohol production of the population R0.

10. A yeast obtained by culturing a yeast strain as claimed in claim 1.

11. The yeast as claimed in claim 10, **characterized in that** the alcohol production thereof is greater than or equal to 95% of the alcohol production of a yeast stemming from the I-4072 strain.

12. The yeast as claimed in claim 10 or 11, **characterized in that** it is in the form of cream yeast, pressed yeast, dry yeast or deep-frozen yeast.

13. The use of a yeast as claimed in one of claims 10 to 12, for producing alcohol.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- The Alcohol Textbook. 2003
- **POWELL ; DIACETIS.** *J. Inst. Brew.,* 2007, vol. 1131 (1), 67-74
- **GRAEME M. WALKER.** Yeast Physiology and Biotechnology. Wiley, 1998, 143
- The Yeasts. Academic Press, 1969, vol. 1
- **G. REED ; T.W. NAGODAWITHANA.** Yeast Technology. Van Nostrand Reinhold, 1991